# EUROPEAN PATENT APPLICATION

(11) **EP 1 854 587 A2**
(43) Date of publication of application: **14.11.2007**
(21) Application number: 07250979.7
(22) Date of filing: 08.03.2007
(51) Int. Cl.: B25B 23/143, A61B 17/88

(54) **Magnetic torque-limiting device and method**

(30) Priority: 12.05.2006 US 799650 P; 31.08.2006 US 513052
(71) Applicant: Teleflex Medical Incorporated, Limerick, PA 19468-1699 (US)
(72) Inventor: Gross, James R., Richmond, IL 60071-9234 (US)
(74) Representative: Hedges, Martin Nicholas

(57) **Abstract**

In a torque-limiting device, a first end engages a fastener, a second end receives an applied torque, and a magnetic torque limiter transmits the applied torque from the second end to the first end to rotate the first end in conjunction with the second end when the applied torque is less than a predetermined limit.

## Description

### CROSS REFERENCE TO RELATED APPLICATIONS

This application claims priority to U.S. Provisional Patent Application Serial No. 60/799,650, filed on May 12, 2006, titled "MAGNETIC TORQUE LIMITING DEVICE AND METHOD," the disclosure of which is incorporated herein by reference in its entirety.

### FIELD OF THE INVENTION

The present invention generally relates to a magnetic torque-limiting device and method. More particularly, the present invention pertains to a device and method for limiting an amount of torque applied by the device.

### BACKGROUND OF THE INVENTION

In various manufacturing, construction, and medical industries, fasteners are utilized that are threaded or screwed into place. These fasteners may include a predetermined amount of torque that has been determined to be optimal for a given fastening situation. In addition, the fastener may include a predetermined amount of torque that has been determined to fatigue or break the fastener. Often, these predetermined torque values are determined by the manufacturer or by a testing facility. In use, a technician or user may employ a device such as a torque-limiting device or torque wrench to set the fastener according to the predetermined amount of torque. In a particular example, bone screws may be employed by surgeons to reconstruct bones or attach replacement components to bones of patients. In such circumstances, applying a proper amount of torque may be critically important.

Conventional torque-limiting devices utilize forces from coil springs and spring washers along with friction to limit the amount of torque applied. Unfortunately, as components within these conventional torque-limiting devices slide by one another, wear may alter the torque setting of the conventional torque-limiting device. As such, conventional torque-limiting devices need to be re-calibrated to maintain their torque limit range.

Conventional torque-limiting device mechanisms may also fail and bind or lockup thereby eliminating the torque-limiting effect and essentially converting the tool to a rigid, non-limiting tool with torque-limiting now regulated to the tool users ability to discern torque forces by hand. This could lead to over-torqueing, an unsafe condition.

An additional aspect of conventional torque-limiting devices is that the torque-limiting event occurs through the same axis as the tool shaft thereby sending shock waves along the tool axis and into the fastener.
Accordingly, it is desirable to provide a device and method capable of overcoming the disadvantages described herein at least to some extent.

### SUMMARY OF THE INVENTION

The foregoing needs are met, to a great extent, by the present invention, wherein in one respect a device and method of limiting an amount of torque applied is provided.

An embodiment of the present invention pertains to a torque-limiting device. The torque-limiting device includes a first end to engage a fastener, a second end to receive an applied torque, and a magnetic torque limiter to transmit the applied torque from the second end to the first end to rotate the first end in conjunction with the second end when the applied torque is less than a predetermined limit.

Another embodiment of the present invention relates to a torque-limiting device. The torque-limiting device includes a shaft, housing, first magnet, second magnet, first lever, and second lever. The shaft includes a distal end to engage a fastener and a proximal end. The shaft defines an axis around which a clockwise rotation vector is aligned towards the distal end. The housing is pivotally secured to the proximal end. The housing includes an outer surface and an inner surface. The inner surface includes at least a first lug protruding radially inwardly into the housing and at least a second lug protruding radially inwardly into the housing. The first magnet is disposed within the housing. The first magnet is secured to the proximal end. The first magnet includes a first bearing surface. The second magnet is juxtaposed in attractive cooperative alignment with the first magnet. The second magnet includes a second bearing surface. The first magnet is drawn towards the second magnet with an attractive magnetic force. The first lever is pivotally secured to the proximal end. The first lever has a first lever lug arm to bear against the first lug. The first lever has a first lever magnet arm to bear against the first bearing surface. A clockwise external torque applied to the outer surface urges the first lug to bear against the first lever lug arm and urges the first lever to pivot clockwise. The first lever magnet arm bears against the first bearing surface to urge the first magnet away from the second magnet in response to the clockwise urging of the first lever lug arm. The second lever is pivotally secured to the proximal end. The second lever has a second lever lug arm to bear against the second lug. The second lever has a second lever magnet arm to bear against the second bearing surface. The clockwise external torque applied to the outer surface urges the second lug to bear against the second lever lug arm and urges the second lever to pivot clockwise. The second lever magnet arm bears against the second bearing surface to urge the second magnet away from the first magnet in response to the clockwise urging of the second lever lug arm and the rotation of the housing relative to the shaft is impeded until the first magnet and second magnet are separated by exceeding the attractive magnetic force between them.

Yet another embodiment of the present invention pertains to a torque-limiting device. The torque-limiting device includes a shaft, housing, first magnet, second magnet, first gear and second gear. The shaft includes a distal end to engage a fastener and a proximal end. The shaft defines an axis of rotation along which a rotation vector is aligned towards the distal end. The housing is pivotally secured to the proximal end. The housing includes an outer surface and an inner surface. The inner surface includes at least a first toothed region having teeth protruding radially inwardly into the housing and at least a second toothed region having teeth protruding radially inwardly into the housing. The first magnet is disposed within the housing. The first magnet is secured to the proximal end. The first magnet includes a first toothed carrier. The second magnet is juxtaposed in attractive cooperative alignment with the first magnet. The second magnet includes a second toothed carrier. The first magnet is drawn towards the second magnet with an attractive magnetic force. The first gear is pivotally secured to the proximal end. The first gear has teeth to engage the first toothed region and the first toothed carrier. A clockwise external torque applied to the outer surface urges the first toothed region to engage the first gear and clockwise rotate the first gear. The clockwise urging of the first gear urges the first magnet away from the second magnet. The second gear is pivotally secured to the proximal end. The second gear has teeth to engage the second toothed region and the second toothed carrier. The clockwise external torque applied to the outer surface urges the second toothed region to engage the second gear and clockwise rotate the second gear. The clockwise urging of the second gear urges the second magnet away from the first magnet and the rotation of the housing relative to the shaft is impeded until the first magnet and the second magnet are separated by exceeding the attractive magnetic force between them.

Yet another embodiment of the present invention relates to a torque-limiting device. The torque-limiting device includes a shaft, first magnetic assembly, housing and second magnetic assembly. The shaft includes a distal end to engage a fastener and a proximal end. The first magnetic assembly is secured to the proximal end. The first magnetic assembly includes a first plurality of magnets. Each one of the first plurality of magnets has a North side adjacent to a South side of another one of the first plurality of magnets. The housing is pivotally secured to the proximal end. The housing includes an outer surface to receive an applied torque. The second magnetic assembly is secured to the housing. The second magnetic assembly includes a second plurality of magnets. Each one of the second plurality of magnets having a North side adjacent to a South side of another one of the second plurality of magnets. Each of the North and South sides of the second magnetic assembly is juxtaposed in attractive cooperative alignment with respective opposite South and North sides, respectively of, the first magnetic assembly to generate an array of attractive magnetic forces between the shaft and the housing.

Yet another embodiment of the present invention pertains to a torque-limiting device. The torque-limiting device includes a shaft, housing, first magnet, second magnet, first gear, and second gear. The shaft includes a distal end to engage a fastener and a proximal end. The shaft defines an axis of rotation along which a rotation vector is aligned towards the distal end. The housing is pivotally secured to the proximal end. The housing includes an outer surface and an inner surface. The inner surface includes at least a first toothed region having teeth protruding radially inwardly into the housing and at least a second toothed region having teeth protruding radially inwardly into the housing. The first magnet is disposed within the housing. The first magnet is secured to the proximal end. The first magnet includes a first toothed carrier. The second magnet is juxtaposed in attractive cooperative alignment with the first magnet. The second magnet includes a second toothed carrier. The first magnet is aligned with the second magnet with an attractive magnetic force between them. The first gear is pivotally secured to the proximal end. The first gear has teeth to engage the first toothed region and the first toothed carrier. A clockwise external torque applied to the outer surface urges the first toothed region to engage the first gear and clockwise rotate the first gear. The clockwise urging of the first gear urges the first magnet to laterally slide out of alignment with the second magnet. The second gear is pivotally secured to the proximal end. The second gear has teeth to engage the second toothed region and the second toothed carrier. The clockwise external torque applied to the outer surface urges the second toothed region to engage the second gear and clockwise rotate the second gear. The clockwise urging of the second gear urges the second magnet to laterally slide out of alignment with the first magnet and wherein rotation of the housing relative to the shaft is impeded until the first magnet and the second magnet laterally slide past each other by exceeding the attractive magnetic force between them.

Yet another embodiment of the present invention relates to a torque-limiting device. The torque-limiting device includes a shaft, housing, first magnet, second magnet and lever. The shaft includes a distal end to engage a fastener and a proximal end. The shaft defines an axis of rotation along which a rotation vector is aligned towards the distal end. The housing is pivotally secured to the proximal end. The housing includes an outer surface and an inner surface. The inner surface includes at least one lug protruding radially inwardly into the housing. The first magnet is disposed within the housing. The first magnet is secured to the proximal end. The second magnet is slidably secured to the proximal end and juxtaposed in attractive cooperative alignment with the first magnet. The second magnet includes a magnet bearing surface. The first magnet is urged towards alignment with the second magnet with an attractive magnetic force. The lever is pivotally secured to the proximal end. The lever has a lug bearing surface to bear against the lug. The lever has an arm to bear against the magnet bearing surface. A clockwise external torque applied to the outer surface urges the lug to bear against the lug bearing surface and urges the lever to pivot clockwise. The arm bears against the magnet bearing surface and urges the second magnet out of alignment from the first magnet in response to the clockwise urging of the arm and the rotation of the housing relative to the shaft is impeded until the first magnet and the second magnet laterally slide past each other by exceeding the attractive magnetic force between them.

Yet another embodiment of the present invention pertains to a torque-limiting device. The torque-limiting device includes a shaft, first magnetic assembly, housing, and second magnetic assembly. The shaft includes a distal end to engage a fastener and a proximal end. The first magnetic assembly secured to the proximal end. The first magnetic assembly includes a first plurality of magnets. The housing is pivotally secured to the proximal end. The housing includes an outer surface to receive an applied torque and the housing includes an inner surface. The inner surface includes at least one lug protruding radially inwardly into the housing. The second magnetic assembly is pivotally secured to the proximal end. The second magnetic assembly includes a second plurality of magnets, an axis about which the second magnetic assembly rotates, and a lip offset from the axis. The second magnetic assembly is juxtaposed in attractive cooperative alignment with the first magnetic assembly to generate an attractive magnetic force between them. The rotation of the housing relative to the shaft urges the lug to bear upon the lip and rotate the second magnetic assembly away from and out of alignment with the first magnetic assembly. The rotation of the housing relative to the shaft is impeded until the separation of the first magnetic assembly and the second magnetic assembly in response to exceeding the attractive magnetic force between them.

Yet another embodiment of the present invention relates to a torque-limiting device includes a shaft, housing, race, first magnet, and second magnet. The shaft includes a distal end to engage a fastener and a proximal end. The shaft defines an axis of rotation along which a rotation vector is aligned towards the distal end. The housing is pivotally secured to the proximal end. The housing includes an outer surface and an inner surface. The inner surface includes at least one lug protruding radially inwardly into the housing. The race is secured to the proximal end. The race is oriented perpendicularly relative to the shaft. The first magnet is slidably disposed within the race. The first magnet includes a bearing surface to bear against the lug. The second magnet is slidably disposed within the race and juxtaposed in repulsive cooperative alignment with the first magnet. The first magnet is repulsed from the second magnet with a repulsive magnetic force that urges the bearing surface against the lug to impede rotation of the housing relative to the shaft. An applied torque to the outer surface urges the lug to bear against the bearing surface and urges the first magnet towards the second magnet and the rotation of the housing relative to the shaft is impeded until the applied torque exceeds a predetermined limit.

Yet another embodiment of the present invention pertains to a torque-limiting device. The torque-limiting device includes a shaft means, handle means, and magnetic limiting torque means. The shaft means engages a fastener. The handle means receives an applied torque. The magnetic torque-limiting means couples the shaft means and handle means and magnetically limiting an amount of torque externally applied to the handle means from being transmitted to the fastener. The magnetic torque-limiting means is configured to transmit the applied torque from the handle means to the shaft means in response to the applied torque being less than a predetermined torque limit.

Yet another embodiment of the present invention relates to a torque meter. The torque meter includes a cam and a magnetic coupling. The cam receives an applied torque. The magnetic coupling includes a pair of magnetically active components that are urged together by an attractive magnetic force. The cam is configured to urge the pair of magnetically active components apart in response to the applied torque.

Yet another embodiment of the present invention pertains to a torque meter. The torque meter includes a first magnet, cam and second magnet. The cam receives an applied torque. The second magnet is juxtaposed in repulsive cooperative alignment with the first magnet. The cam is configured to urge the first magnet towards the second magnet in response to the applied torque.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1 is a perspective view of the torque-limiting device according to an embodiment of the invention.

FIGS. 2A-2D are diagrams illustrating a variety of manners of employing magnets to limit an amount of torque applied according to various embodiments of the invention.

FIG. 3A is a top view of a torque-limiting device in a first position according to the magnetic configuration shown generally in FIG. 2A.

FIG. 3B is a top view of a torque-limiting device in a second position according to the magnetic configuration shown generally in FIG. 2A.

FIG. 4A is a top view of another torque-limiting device in a first position according to the magnetic configuration shown generally in FIG. 2A.

FIG. 4B is a top view of another torque-limiting device in a second position according to the magnetic configuration shown generally in FIG. 2A.

FIG. 5A is a top view of the torque-limiting device.

FIG. 5B is a perspective view of the torque-limiting device shown in FIG. 5A.

FIG. 6A is an exploded view of a pair of magnetic disks suitable for use with the torque-limiting device.

FIG. 6B is a cutaway view of another torque-limiting device.

FIG. 7 is a top view of another torque-limiting device.

FIG. 8A is a top view of a torque-limiting device.

FIG. 8B is a perspective view of the torque-limiting device in FIG. 8A.

FIG. 9A is an exploded view of a torque-limiting device according to a magnetic configuration shown generally in FIG. 2C.

FIG. 9B is a top view of the torque-limiting device shown in FIG. 9A in a first position.

FIG. 9C is another top view of the torque-limiting device of FIG. 9A in a second position.

FIG. 10 is an exploded view of another torque-limiting device.

FIG. 11 is a simplified top view of a torque sensing device.

FIG. 12 is a top view of another torque sensing device.

FIG. 13 is a perspective view of the torque sensing device of FIG. 12.

FIG. 14A is a cross sectional view A-A of the torque-limiting device in a first torque-limiting configuration.

FIG. 14B is a cross sectional view A-A of the torque-limiting device in a second torque-limiting configuration.

### DETAILED DESCRIPTION

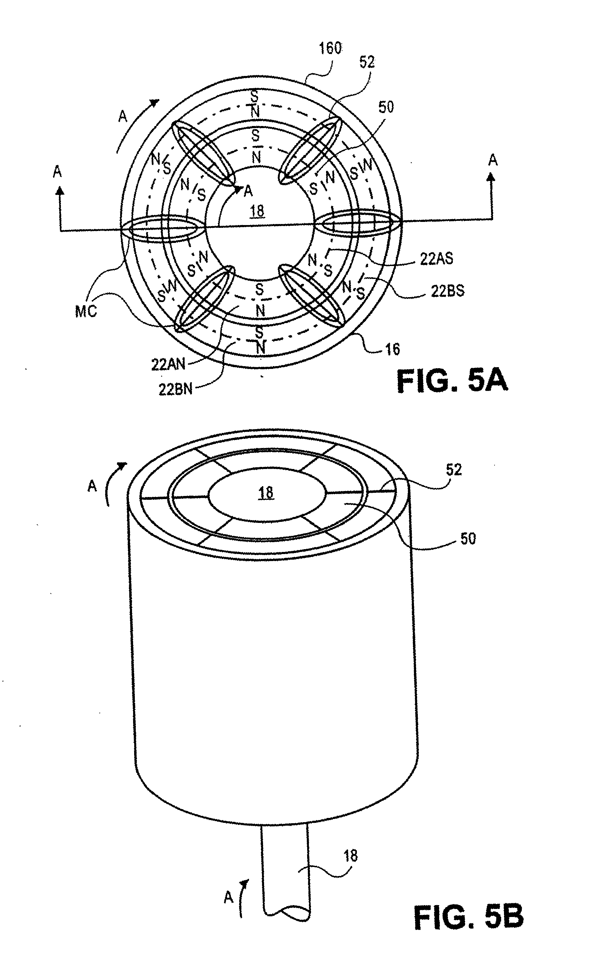

The present invention provides a magnetic torque-limiting device and method. A detailed description of the magnetic method utilized in the preferred embodiment will clarify the understanding of the phenomenon of magnetic coupling and the magnetic circuit that is created in the preferred embodiment. The individual magnets are made from a rare earth material, such as Samarium Cobalt, SmCo, or Neodymium Iron Boron NdFeB, the former being preferred over the later. NdFeB, due to its iron component, will rust, SmCo contains no iron and will not rust. The shaft and tube are made from a magnetically conductive stainless steel, such as T410 Smls Annealed or similar magnetically conductive materials. The shaft assembly includes magnets connected to the shaft. They are arranged so that the separate magnetic elements, i.e. the magnets are in a mutually magnetically attractive arrangement. The magnetic lines of force pass from one magnet, through the shaft and into the adjacent magnet; opposite poles adjacent to and attracting each other. See FIG. 5A.

A similar arrangement is made between the tube and the tube magnets with magnetic lines of force traveling from magnet to magnet through the tube.

When the shaft is positioned inside the tube the north magnets on the shaft will align with the south magnets on the tube, opposite poles attracting. This arrangement is done by design. The lines of force that flow through the shaft magnets and shaft will join with the lines of magnetic force flowing through the tube and tube magnets. These lines of force form a complete circle or closed loop and as such are called a magnetic circuit.

The magnetic relationship between shaft and tube is static with neither part seeking a different alignment. The number of magnets used in the preferred embodiment permits the creation of three complete magnetic circuits. These three magnetic circuits magnetically couple the tube to the shaft. When the tube is used as a handle and rotated the shaft will follow. However, the magnetic force between the tube and shaft is finite and pre-established by its design.

When the shaft is connected to a fastener, rotation of the tube or handle will force the shaft to rotate thereby installing the fastener. However, when the resistance on the fastener exceeds the magnetic force between the tube and shaft the shaft will stop rotating but the tube will continue to rotate as long as turning forces are applied that exceed the magnetic force. When this occurs the magnetic circuit is first distorted and then breaks as the tube magnets rotate to re-align with the next set of shaft magnets and create the next set of magnetic circuits. The circuit is broken when the turning force exceeds the magnetic force. However, the turning force has no effect on the magnetic force; that force is predetermined in the design of the magnetic circuit.

The peak magnetic force is achieved when the circuit is complete and the lines of force are not being distorted. The moment the magnets are moved relative to one another the lines of force become distorted and the magnetic attraction begins to lessen. This is an inherent safety feature of the design in that the predetermined force cannot be exceeded but only lessened. Furthermore, the magnetic force has no mechanical dependencies and as such does not require recalibration or maintenance.

In one example of a preferred embodiment, the torque-limiting device is utilized to install fasteners into a bone of a patient. Bones varies in density from surface to core and this presents a number of difficulties with regard to fastening. Primary among these difficulties are the inherent hardness of the bone outer surface and the varying density of the bones core. To install a fastener in bone generally calls for a quantifiable torque force. This torque force limit is established by the fastener manufacturer and is usually expressed as a "not to exceed" value. (Excessive torque can break the fastener). Torque limiting devices are typically made to meet but not exceed the proscribed torque value. When installing a fastener, applying excessive torque may have negative consequences. It is an advantage of embodiments of the invention that the magnetic torque-limiting device substantially and/or continually maintains a pre-set, non exceedable, torque limit. It is another advantage that this pre-set torque limit is essentially unaffected by frictional wear, as the working components do not touch one another. As such, the need for testing and recalibration of the torque-limiting device may be eliminated. It is yet another advantage of embodiments that the magnetic torque-limiting device may reduce or eliminate shock loads upon the fastener and/or patient. This is a result of the non-parallel relationship of the axis of magnetic torque-limiting and the axis of the tool. In contrast, a conventional torque-limiting device may generate shock loads that may break or damage the fasteners. These shock loads can be attributed to the mechanical nature of current tools, their actions are abrupt mechanical movements, with metal to metal components snapping into detents with force and considerable speed. The action of the magnetic assembly is one of smooth rotation and realignment without the necessity of mechanical components snapping into place. As described herein, suitable magnets may be utilized to generate attractive and/or repulsive forces that are used to predetermine and limit the amount of torque which that can be applied.

An advantage of magnetically induced torque is that the specific magnetic force, or torque limit, is relatively stable, cannot in and may remain essentially the same over the lifetime of the torque-limiting device.

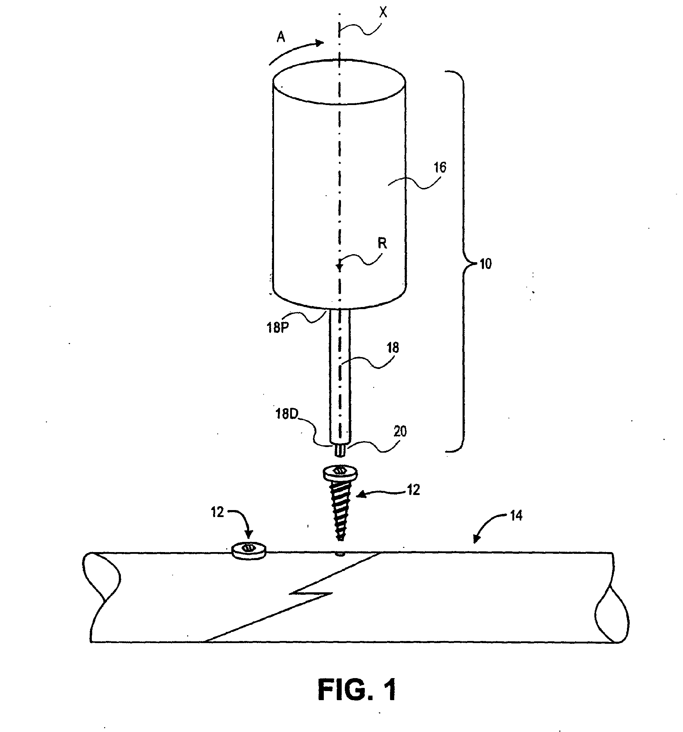

Preferred embodiments of the invention will now be described with reference to the drawing figures, in which like reference numerals refer to like parts throughout. As shown in FIG. 1, a torque-limiting device 10 may be utilized to install a fastener 12 in a substrate 14. The torque-limiting device 10 includes a handle 16 for the user, technician, or surgeon to grasp. The torque-limiting device 10 further includes a shaft 18 connecting the handle 16 to a head 20. The head 20 is configured to engage the fastener 12. In various embodiments, the head 20 and shaft 18 may be essentially a single component or the head 20 may be releasably attached to the shaft 18 to facilitate exchanging the head 20 with another head 20. In general, the shaft engages the handle at a proximal end 18P and engages the fastener 12 at a distal end 18D.

In general, the torque-limiting device 10 is configured such that in response to turning the handle 16 in direction A, an applied torque will urge the shaft 18 to rotate in a similar fashion. The shaft 18 defines an axis X around which a clockwise rotation vector R is aligned towards the distal end 18D of the shaft 18. In general, the rotation vector R is defined as a direction and magnitude of the torque applied to the handle 16 (applied torque) as the applied torque is transmitted along an axis X towards the distal end 18D (the point of application of the force). Typically, to install the fastener 12, a clockwise rotation is applied to the fastener (from a view in-line with the rotation vector R).

The torque-limiting device 10 is configured such that at a threshold or max torque, the shaft 18 ceases to rotate in direction A in response to rotating the handle 16 in direction A. In general, the maximum torque can be defined as the maximum magnetic attraction between the two magnetic assemblies. Once established by the design of the magnetic components, that force cannot be exceeded. In conventional devices, the limit at this threshold torque is affected by frictional forces of one member sliding against another. Unfortunately, this friction results in mechanical wear that may alter the characteristics of the torque applied through the device. It is an advantage of the various embodiments of the magnetic torque-limiting device that friction is a substantially negligible component of the applied torque. As such, the embodiments are essentially unaffected by frictional or mechanical wear.

In addition, while not explicitly shown in FIG. 1, the torque-limiting device 10 may include a display to display the amount of torque being applied to the fastener 12. Furthermore, the torque-limiting device 10 may include a ratcheting mechanism to provide unidirectional or selectable unidirectional rotation of the shaft 18. This is an optional feature so that the operator does not have to change or reset the hand position on the tool.

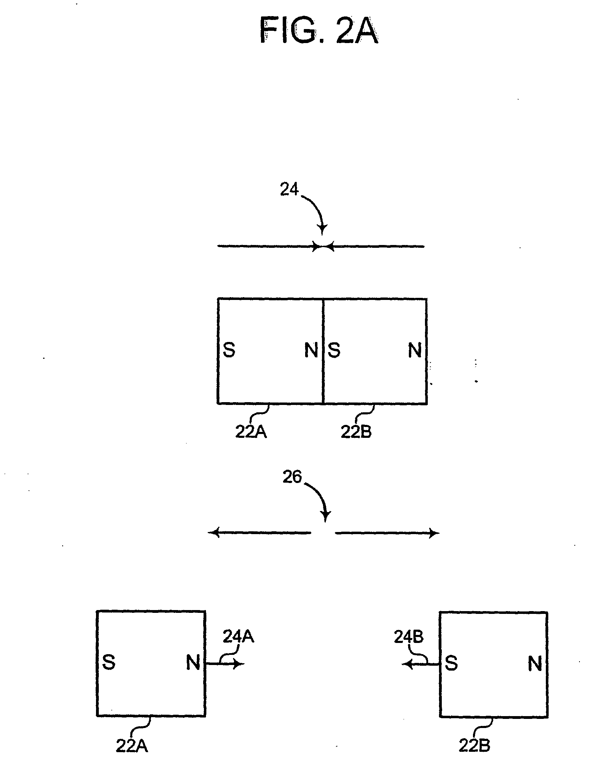

FIGS. 2A-2D are diagrams illustrating a variety of manners of employing magnets to limit an amount of torque applied according to various embodiments of the invention. The magnets include a north sector or north pole and a south sector or pole. These sectors or poles may be configured to coincide with one or more faces. By disposing the respective sectors or poles of the magnets in cooperative alignment, the resulting magnetic force (attractive and/or repulsive) may be utilized in the torque-limiting device.

Two magnets may be brought into attachment by disposing the north sector of one magnet in close proximity to the south sector of the other magnet. As shown in FIG. 2A, a pair of magnets 22A and 22B are positioned in a mutually attractive, 'North' to 'South' arrangement. In this manner, the opposite poles of the respective magnets may be allowed to attract one another. That is, the magnets 22A and 22B are arranged such that their respective magnetic fields are aligned in a North facing South arrangement. Such an arrangement generates an attractive force 24 that is inversely proportional to a square of the distance between the magnets 22A and 22B. In addition, the strength of the attractive force 24 is proportionally related to a sum of the respective strengths of the magnets 22A and 22B. The respective strengths of the magnets are based upon their respective masses, material compositions and the level or degree to which the magnetic material is magnetized. In general, all other factors being equivalent, pulling two magnets apart along a straight line axis requires a relatively greater amount of force as compared to sliding, twisting, or tipping magnets apart. In this regard, the combined attractive force 24 resists a separating force 26 until the separating force 26 exceeds the attractive force 24. As shown in FIG. 2A, the attractive force 24 may include an attractive force 24A and 24B contributed by magnets 22A and 22B, respectively.

In various embodiments, the magnets 22A and 22B may include any suitable magnets, magnetic devices, and/or magnetically attractive material. Suitable magnets may include, for example, permanent magnets, dipoles, electromagnets, and the like. Specific examples of permanent magnets include ferrous or ferrite magnets, rare earth magnets, Neodymium magnets such as Neodymium Iron Boron ("NdFeB"), Samarium-Cobalt ("SmCo") magnets, ceramic magnets, alnico magnets, and the like. Furthermore, the magnets 22A and 22B may include a magnetic intensifier such as an iron or iron bearing cup or shaft. Moreover, the magnets 22A and/or 22B may include any suitable magnetically active material. For the purpose of this disclosure, magnetically active materials include magnets, magnetically inducible materials such as iron, materials with relatively high permeability, and the like.

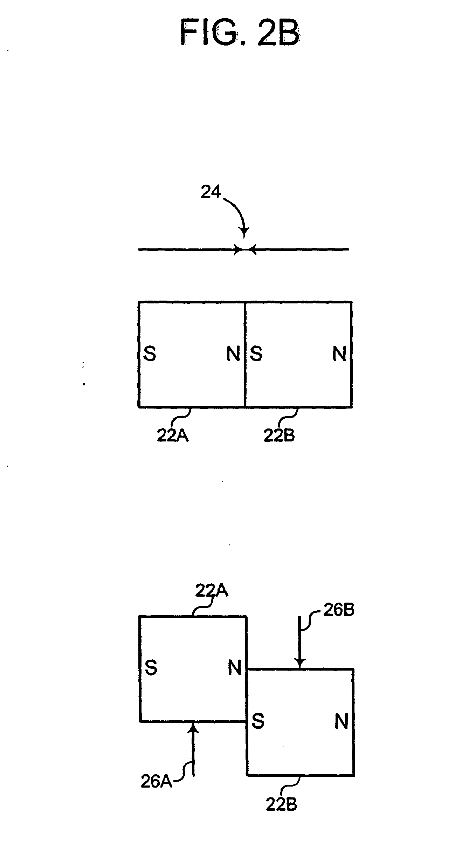

As shown in FIG. 2B, the attractive force 24.may be utilized to resist sliding in response to the separating force perpendicular to the attractive force and composed of a separating force components 26A and 26B. In general, the magnets 22A and 22B may be separated relatively more easily by sliding as compared to pulling as shown in FIG. 2A. A variation of this sliding action may include sliding about an axis or twisting.

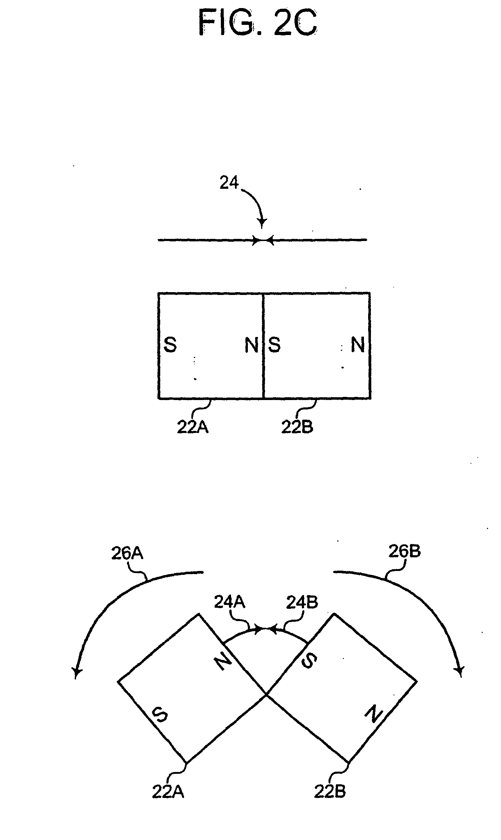

As shown in FIG. 2C, the attractive force 24A and 24B may resist "tipping" of the magnets 22A and 22B by the separation forces 26A and 26B. In general, the magnets 22A and 22B may be separated relatively more easily by tipping as compared to pulling as shown in FIG. 2A.

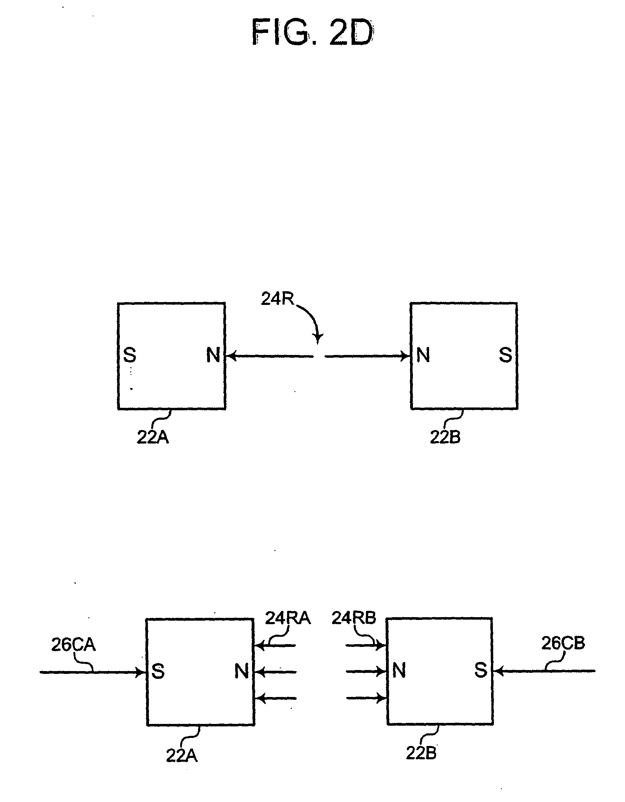

As shown in FIG. 2D, in response to cooperatively aligning the magnets 22A and 22B such that the same poles are facing one another, the magnetic fields generate a repulsive force 24R instead of the attractive force 24. In an embodiment, compressive forces 26CA and 26CB may be configured to urge the magnets 22A and 22B together against repulsive forces 24RA and 24RB.

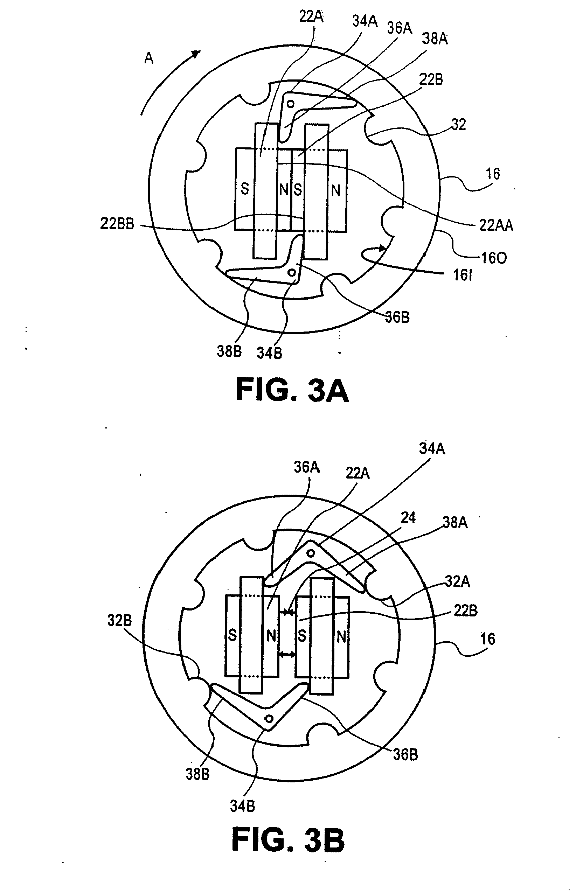

FIG. 3A is a top view of a torque-limiting device in a first configuration utilizing the magnetic orientations shown in FIG. 2A. As shown in FIG. 3A, the magnets 22A and 22B are disposed in cooperative alignment with opposite poles facing one another. In response to rotating the handle 16 in direction A, one or more lugs 32 is brought to bear against one or more levers 34. The levers 34 includes an arm 36 to urge the magnets 22A and 22B apart. The lever 34 further includes an arm 38 to bear against a lug 32. The handle 16 will freely rotate about the tool shaft 18 but for the resistance to turning provided by the magnetic assembly. The magnetic assembly is attached to the tool shaft and is covered by the handle. The handle is designed to physically interfere with the magnetic assembly. Handle lug 32 bears upon lever arm 34 which, in turn, bears upon the magnetic assembly of 22A and 22B. As long as the magnetic force between the magnetic elements is not exceeded, the handle will drive the magnetic assembly and the associated shaft around the tool axis. Typically, this is the fastener application direction and the direction in which torque-limiting is desired as a means of safety.

When the force acting on the magnetic assembly and its associated shaft, induced by the handle lug(s) exceeds the predetermined magnetic force or torque value, the magnets will separate, by means of the levers, and the torque force exerted on the tool shaft will diminish. This is the moment of torque-limiting. The predetermined torque force is reached but not exceeded and the fastener is prevented from being over-torqued.

By varying the length of the arm 36 compared to the arm 38, the force required to separate the magnets may be altered. For example, by increasing the length of the arm 38 as compared to the arm 36, the separating force may be reduced for a given amount of torque placed upon the handle 16. In a particular example, the magnets 22A and 22B and the lever 34 are held in a fixture that is securely attached to the shaft 18 of the torque-limiting device. One or both of the magnets 22A and 22B are free to move away from the other magnet along a path described by the common axis running through both magnets 22A and 22B. In response to an amount of torque being applied to the handle 16, the magnets 22A and 22B are levered apart by the lever 34, as shown in FIG 3B, and the lug 32 slides past the lever 34. Once past the lug 32, the lever 34 returns to its initial, starting position and the magnets 22A and 22B return to their initial or starting position shown in FIG. 3A. In this manner, a limited amount of torque is applied to the shaft 18 by the handle 16. By varying the magnetic strength of the magnets 22A and 22B, the limiting torque/max may be modulated. By redesigning the lever arm 34 to increase the length of the "effort" arm and decrease the length of the "load arm, the force required to move the lever will be reduced.

Therefore, the embodiment shown in FIGS. 3A and 3B include the torque-limiting device 10, shaft 18, housing or handle 16, first magnet 22A, second magnet 22B, first lever 34A, and second lever 34B. The shaft includes the distal end 18D or head 20 to engage the fastener 12 and proximal end 18P. (each shown in FIG. 1) The shaft 18 defines the axis X around which the clockwise rotation vector R is aligned towards the distal end 18D. The housing 16 is pivotally secured to the proximal end 18P. The housing 16 includes an outer surface 160 and an inner surface 161. The inner surface 161 includes at least a first lug 32A protruding radially inwardly into the housing 16 and at least a second lug 32B protruding radially inwardly into the housing 16. The first magnet 22A is disposed within the housing 16. The first magnet 22A is secured to the proximal end 18P. The first magnet 22A includes a first bearing surface 22AA. The second magnet 22B is juxtaposed in attractive cooperative alignment with the first magnet 22A. The second magnet 22B includes a second bearing surface 22BB. The first magnet 22A is drawn towards the second magnet 22B with an attractive magnetic force 24. The first lever 34A is pivotally secured to the proximal end 18P. The first lever 34A has a first lever lug arm 38A to bear against the first lug 32A. The first lever 34A has a first lever magnet arm 36A to bear against the first bearing surface 22AA. A clockwise external torque applied to the outer surface 160 urges the first lug 32A to bear against the first lever lug arm 38A and urges the first lever 34 to pivot clockwise. The first lever magnet arm 36A bears against the first bearing surface 22AA to urge the first magnet 22A away from the second magnet 22B in response to the clockwise urging of the first lever lug arm 38A. The second lever 34B is pivotally secured to the proximal end 18P. The second lever 34B has a second lever lug arm 38B to bear against the second lug 32B. The second lever 34B has a second lever magnet arm 36B to bear against the second bearing surface 22BB. The clockwise external torque applied to the outer surface 160 urges the second lug 32B to bear against the second lever lug arm 38B and urges the second lever 34B to pivot clockwise. The second lever magnet arm 36B bears against the second bearing surface 22BB to urge the second magnet 22B away from the first magnet 22A in response to the clockwise urging of the second lever lug arm 38B and the rotation of the housing 16 relative to the shaft 18 is impeded until the first magnet 22A and second magnet 22B are separated by exceeding the attractive magnetic force 24 between them.

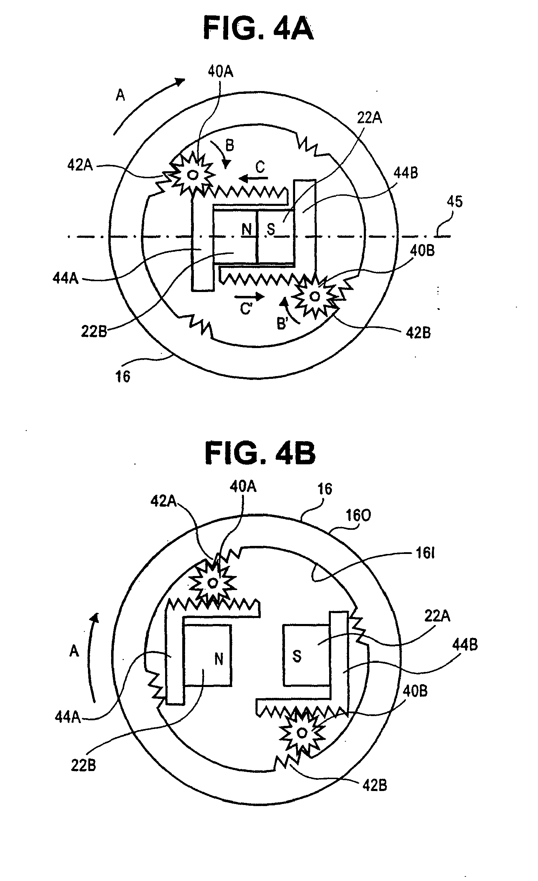

FIG. 4A is a top view of the torque-limiting device in a first configuration again utilizing the magnetic orientation shown in FIG. 2A. In various embodiments, the torque-limiting device may include one or more gears, toothed regions, and the like. In the particular example shown in FIG. 4A, the torque-limiting device 10 includes a pair of gears 40A and 40B configured to mesh with a toothed region 42A and 42B. In addition, the gears 40 are configured to mesh with corresponding toothed assemblies 44A and 44B. The toothed assemblies 44A and 44B are configured to retain the magnets 22A or 22B and are securely attached to the shaft 18 in such a manner so as to allow the magnets 22A and 22B to separate along an axis 45. In operation, as the handle 16 is rotated in direction A and torque is applied to the shaft 18, the gear 40A is caused to mesh with the toothed region 42A and the gear 40A is rotated in direction B. In response to rotating in direction B, the gear 40A urges the toothed assembly 44A in direction C.

Similarly, the gear 40B may mesh with the toothed region 42B and urge the toothed assembly 44B in direction C'. In this manner, the magnets 22A and 22B are urged apart, as shown in FIG. 4B (Separation exaggerated for emphasis). In response to torque being applied to the shaft 18 from the handle 16, as long as the torque limit is not exceeded, the shaft 18 will rotate with the handle 16. In response to this applied torque exceeding the torqueₘₐₓ, the magnets 22A and 22B separate and thereby allow the gear 40A and 40B to advance along the toothed assemblies 44A and 44B. This advancement of the gears 40A and 40B further results in the gears 40A and 40B advancing along the toothed regions 42A and 42B. In response to the gears 40A and 40B advancing past the toothed regions 42A and 42B, the gears 40A and 40B may freely rotate to allow the magnets 22A and 22B to attract one another again as illustrated in FIG 4A.

Therefore, the embodiment shown in FIGS. 4A and 4B include the torque-limiting device 10, shaft 18, housing 16, first magnet 22A, second magnet 22B, first gear 40A and second gear 40B. The shaft 18 includes a distal end 18D to engage a fastener 12 and a proximal end 18P. The shaft 18 defines an axis of rotation X along which a rotation vector R is aligned towards the distal end 18D. The housing 16 is pivotally secured to the proximal end 18P. The housing 16 includes an outer surface 160 and an inner surface. The inner surface 16I includes at least a first toothed region 42A having teeth protruding radially inwardly into the housing 16 and at least a second toothed region 42B having teeth protruding radially inwardly into the housing 16. The first magnet 22A is disposed within the housing 16. The first magnet 22A is secured to the proximal end 18P. The first magnet 22A includes a first toothed carrier 44A. The second magnet 22B is juxtaposed in attractive cooperative alignment with the first magnet 22A. The second magnet 22B includes a second toothed carrier 44B. The first magnet 22A is drawn towards the second magnet 22B with an attractive magnetic force 24. The first gear 40A is pivotally secured to the proximal end 18P. The first gear 40A has teeth to engage the first toothed region 42A and the first toothed carrier 44A. A clockwise external torque applied to the outer surface 160 urges the first toothed region 42A to engage the first gear 40A and clockwise rotate the first gear 40A. The clockwise urging of the first gear 40A urges the first magnet 22A away from the second magnet 22B. The second gear 40B is pivotally secured to the proximal end 18P. The second gear 40B has teeth to engage the second toothed region 42B and the second toothed carrier 44B. The clockwise external torque applied to the outer surface 160 urges the second toothed region 42B to engage the second gear 40B and clockwise rotate the second gear 40B. The clockwise urging of the second gear 40B urges the second magnet 22B away from the first magnet 22A and the rotation of the housing 16 relative to the shaft 18 is impeded until the first magnet 22A and the second magnet 22B are separated by exceeding the attractive magnetic force 24 between them.

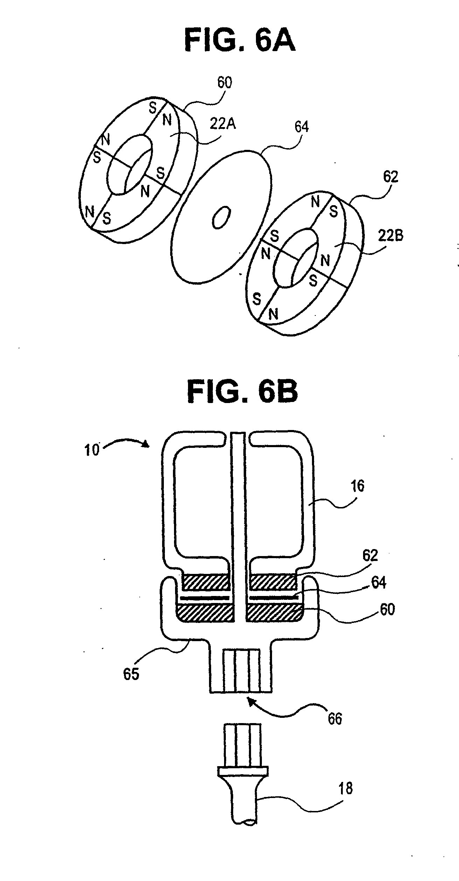

FIG. 5A is a top view of the torque-limiting device 10 according to FIG. 2B. In general, the torque-limiting device 10 according to the embodiment of FIG. 2B pertains to the juxtaposition of at least two magnetic elements but preferentially, refers to a sufficient number of magnets to complete a circular array. The magnetic elements are aligned by opposite poles that attract each other. Lines of magnetic force flow from one magnet to the next when opposite poles are aligned. The lines of force seek to complete a magnet circuit so that no energy is lost to space. Four adjacent and aligned magnets of opposite polarity will create a circuit. The force required to separate all the magnetic circuts contained in a magnetic array determines the amount of torque that can be applied through the device. In general, the embodiments shown in FIGS. 5A, 5B, 6A, and 6B include assemblies of magnets in cooperative alignment that generate an array of magnetic interactions. While the embodiments illustrated depict a circular array, in other embodiments, the array may be arcuate, linear, or the magnets may be arranged in a repulsive relationship.

As shown in FIG. 5A, the torque-limiting device 10 includes a shaft magnet assembly 50 and a handle magnet assembly 52. The shaft magnet assembly 50 is secured to a shaft 18 and includes at least two magnets such as the magnet 22AS, and 22AN. In a particular example, the shaft magnet assembly 50 includes six magnets, 3 of 22AN and 3 of 22AS arranged in an alternating circular array about the shaft 18. The handle magnet assembly 52 includes at least two corresponding magnets such as the magnet 22BS, and 22BN. In a particular example, at least two corresponding magnets of 22BS, and 22BN of the handle magnet assembly 52 are secured to the handle 16. The tool shaft 18 and handle 16 are made from magnetically conductive materials. The use of these materials permits the magnetic lines of force to become a complete magnetic circuit ("MC"). As shown in FIG. 5A, the interaction of each of the magnets in the shaft magnet assembly 50 with the corresponding magnet of the handle magnet assembly 52 generates a magnetic circuit. The magnetic circuit controls the rotation of the shaft 18 relative to the rotation of the handle 16 with the strength of the sum total of all of the magnetic circuits created by the magnetic array.

An air gap separates the shaft magnet assembly 50 from the handle magnet assembly 52. To increase the magnetic force between the shaft magnetic assembly and the handle magnetic assembly, this gap may be made as small as possible given the available manufacturing tolerances. In a particular example, the shaft magnet assembly 50 and/or the handle magnet assembly 52 may be machined in a rounded manner. As such, the gap may be reduced to one or a few thousandths of an inch. This gap facilitates rotation of the shaft magnet assembly 50 with respect to the handle magnet assembly 52. In this manner, in response to an applied torque exceeding the ability of the magnets to remain in attractive alignment, the handle 16 may rotate without also rotating the shaft 18. The rotation of handle 16 also separates or breaks the magnetic circuit between the two corresponding magnets thereby reducing the magnets mutual attractive force, creating the torque limit of the tool.

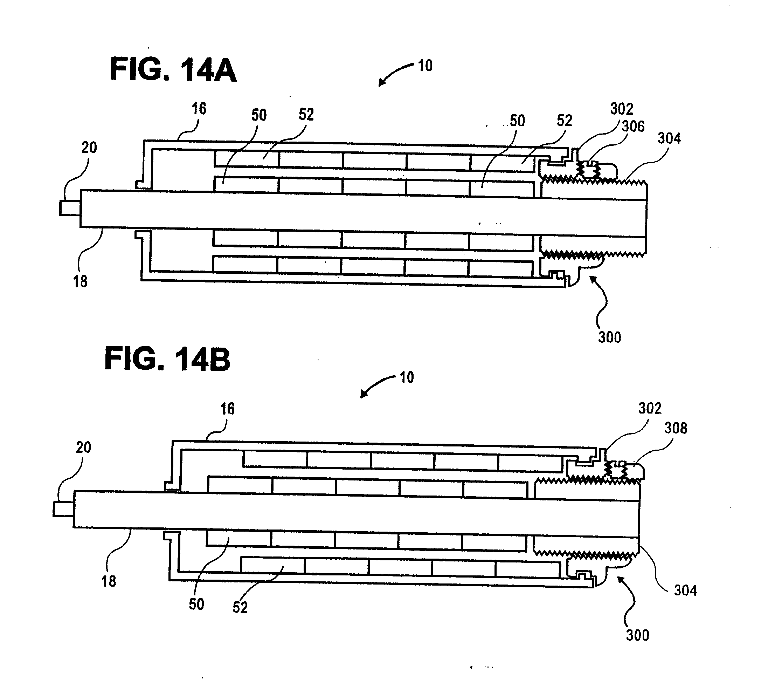

To adjust the torque limit of the torque-limiting tool 10, the shaft magnet assembly 50 may be axially displaced relative to the handle magnet assembly 52. In this regard, FIGS. 14A and 14B show a cross sectional view A-A in accordance with an embodiment of the invention. In FIGS. 14A and 14B, an example of a manner of adjusting the torque limit by axially displacing the shaft magnet assembly 50 relative to the handle magnet assembly 52 is shown.

As shown in FIG. 5B, turning the handle 16 causes the handle magnet assembly 52 to urge the shaft magnet assembly 50 to rotate in the same direction and thereby rotates the shaft 18. In this regard, the shaft magnet assembly 50 are magnetically linked to the handle magnet assembly 52 and as such are driven by the handle 16. However, in this and other embodiments, there may be essentially no physical connection between the handle 16 and shaft 18. In response to the torque applied to the handle 16 exceeding the maximum amount of torque which can be translated to the shaft, the shaft 18 may cease to rotate in direction "A" regardless of the action of the handle 16. That is, the torque limit has been exceeded and the magnetic element alignment is separated or broken. If the handle 16 continues to turn, the magnets realign to the next circuit alignment.

In addition, portions of the tool shaft 18 and the handle 16 which contact the magnets may be made from magnetically conductive materials. The use of these materials permits the magnetic lines of force to become a complete magnetic circuit. In a particular example, suitable conductive materials include steel and magnetically permissive stainless steels such as type 440 series stainless steel, series 630/17-4 cond.A stainless steel, and the like.

FIG. 6A is an exploded view of a pair of magnetic disks 60 and 62 suitable for use with the torque-limiting device operating using the magnetic alignment configuration of FIG. 2B. As shown in FIG 6A, the magnetic disks 60 and 62 are similar to the shaft magnet assembly 50. In general, the magnetic orientation of the magnetic disks 60 and 62 compliment one another and the magnetic disks 60 and 62 are in a mutually magnetic attracted relationship, opposite poles facing along the common axis. That is, when brought into alignment and relatively close proximity, the magnetic disks 60 and 62 attract one another. Also shown in FIG. 6A, an optional slip sheet or divider disk 64 may be disposed between the magnetic disks 60 and 62 to reduce friction between rotating components, for example. Materials suitable for use as the divider disk 64 include relatively low friction material, metals, and the like. In particular, stainless steel, steel, graphite, ultra high molecular weight polyethylene, Teflon^{™}, and the like may be suitable for use as the divider disk 64. The use of ball or roller bearings to separate the magnets and permit low friction movement is also anticipated.

FIG. 6B is a cutaway view of a torque-limiting device utilizing the magnetic disks shown in FIG. 6A. As shown in FIG. 6B, the torque-limiting device includes a body 65 that may be secured to a shaft 18. To secure the shaft 18, the body 65 optionally includes a socket 66 to mate with the shaft 18. If included, the socket 66 facilitates exchanging the shaft 18 with new or different shafts.

The magnetic disk 60 is secured to the body 65 and the magnetic disk 62 is secured to the handle 16. The handle 16 and the body 65 are secured to one another in a freely rotating manner. By turning the handle 16, the magnetic disk 62 urges the magnetic disk 64 to rotate in a similar fashion. In operation, when the resistive force on a fastener coupled to the shaft 18 exceeds the magnetic attraction ability between the magnetic disks 60 and 62, then the handle will began to distort the magnet fields attraction causing a reduction in magnetic attraction and a comparable reduction of the torque capable of being applied to the fastener 12. Once the magnetic field separation has occurred the magnets are permitted to realign for the next torque-limiting event.

In addition, embodiments of the invention may include multiple magnetic disks 60 and 62. For example, any suitable number of magnetic disks may be stacked along the axis of the torque-limiting device 10 with alternating disks being secured to either the shaft 18 or the handle 16. In this manner, the torqueₘₐₓ may be varied.

Therefore, the embodiments shown in FIGS. 5A, 5B, 6A and 6B include the torque-limiting device 10, shaft 18, first magnetic assembly 50, housing 16 and second magnetic assembly 52. The shaft 18 includes a distal end 18D to engage a fastener 12 and a proximal end 18P. The first magnetic assembly 50 is secured to the proximal end 18P. The first, magnetic assembly 50 includes a first plurality of magnets 22As. Each one of the first plurality of magnets has a North side adjacent to a South side of another one of the first plurality of magnets. The housing 16 is pivotally secured to the proximal end 18P. The housing 16 includes an outer surface 160 to receive an applied torque. The second magnetic assembly 52 is secured to the housing 16. The second magnetic assembly 52 includes a second plurality of magnets 22Bs. Each one of the second plurality of magnets having a North side adjacent to a South side of another one of the second plurality of magnets 22Bs. Each of the North and South sides of the second magnetic assembly 52 is juxtaposed in attractive cooperative alignment with respective opposite South and North sides, respectively of, the first magnetic assembly 50 to generate an array of attractive magnetic forces 24 between the shaft 18 and the housing 16.

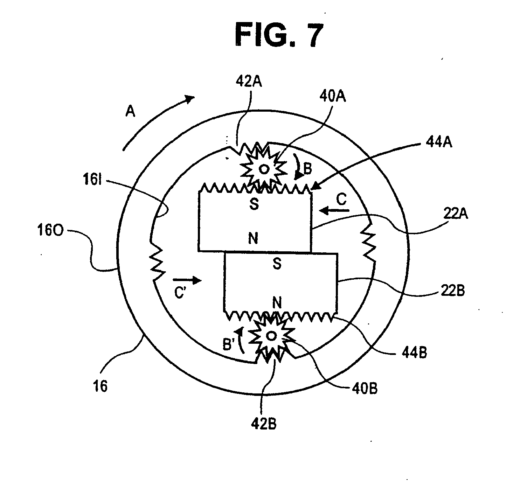

FIG. 7 is a top view of a torque-limiting device utilizing the sliding magnets shown in FIG. 2B. Certain elements of the torque-limiting device of FIG. 7 are similar to the torque-limiting device of FIGS. 4A and 4B and thus, for the purpose of brevity, the description of those elements will not be repeated herein. The gears 40A and 40B and the magnets 22A and 22B are secured to the shaft 18 in such a way so as to allow the gears 40A and 40B to freely rotate and to allow the magnets 22A and 22B to freely slide with respect to one another. As shown in FIG. 7, in response to the handle 16 turning in direction A, the gears 40A and 40B rotate in direction B when respectively engaged by the toothed regions 42A and 42B. As a result, the magnets 22A and 22B are urged to slide as indicated (C and C¹ respectively). The attractive magnetic force exhibited by the magnets 22A and 22B resists this sliding movement and thereby limits the transmitted torque applied to the shaft 18 by the handle 16. Other factors that may contribute to transmission of torque include one or more of magnet material type, size, degree of magnetism, proximity, surface, contact friction, and relative magnet(s) position, relative to one another, and the like. By varying any one or more of these factors, the torqueₘₐₓ may be modulated or controlled. When the torque applied to the handle exceeds the torqueₘₐₓ, the magnets 22A and 22B slide past one another sufficiently to allow the gears 40A and 40B to rotate past the toothed regions 42A and 42B. Once past the toothed regions 42A and 42B, the gears 40A and 40B are no longer urged to rotate in direction B and may freely rotate to allow the magnets 22A and 22B to realign by laterally sliding. In general, the term "sliding" and "lateral sliding" refers to the movement of the first magnet 22A relative to the second magnet 22B irrespective of the absolute direction that the magnets 22A and 22B travel.

Therefore, the embodiments shown in FIG. 7 includes the torque-limiting device 10, shaft 18, housing 16, first magnet 22A, second magnet 22B, first gear 40A, and second gear 40B. The shaft 18 includes a distal end 18D to engage a fastener 12 and a proximal end 18P. The shaft 18 defines an axis of rotation X along which a rotation vector R is aligned towards the distal end 18D. The housing 16 is pivotally secured to the proximal end 18P. The housing 16 includes an outer surface 16O and an inner surface 161. The inner surface 161 includes at least a first toothed region 42A having teeth protruding radially inwardly into the housing 16 and at least a second toothed region 42B having teeth protruding radially inwardly into the housing 16. The first magnet 22A is disposed within the housing 16. The first magnet 22A is secured to the proximal end 18P. The first magnet 22A includes a first toothed carrier 44A. The second magnet 22B is juxtaposed in attractive cooperative alignment with the first magnet 22A. The second magnet 22B includes a second toothed carrier 44B. The first magnet 22A is aligned with the second magnet 22B with an attractive magnetic force 24 between them. The first gear 40A is pivotally secured to the proximal end 18P. The first gear 40A has teeth to engage the first toothed region 42A and the first toothed carrier 44A. A clockwise external torque applied to the outer surface 16O urges the first toothed region 42A to engage the first gear 40A and clockwise rotate the first gear 40A. The clockwise urging of the first gear 40A urges the first magnet 22A to laterally slide out of alignment with the second magnet 22B. The second gear 42B is pivotally secured to the proximal end 18P. The second gear 40B has teeth to engage the second toothed region 42B and the second toothed carrier 44B. The clockwise external torque applied to the outer surface 16O urges the second toothed region 42B to engage the second gear 40B and clockwise rotate the second gear 40B. The clockwise urging of the second gear 40B urges the second magnet 22B to laterally slide out of alignment with the first magnet 22A and wherein rotation of the housing 16 relative to the shaft 18 is impeded until the first magnet 22A and the second magnet 22B laterally slide past each other by exceeding the attractive magnetic force 24 between them.

FIG. 8A is a top view of another torque-limiting device utilizing a sliding magnet arrangement as in FIG. 2B. As shown in FIG. 8A, the handle 16 includes one or more bars or ridges 70 configured to urge a lever 72 in direction B in response to the handle being rotated in direction A. Rotation of the lever 72 urges the magnet 22B in direction C. This sliding motion is opposed by the magnetically attractive force between the magnets 22A and 22B. The magnets 22A and 22B and the lever 72 are secured to the shaft 18. As such, the magnetic resistance to sliding facilitates the transfer of torque from the turning handle 16 to the shaft 18. Once the applied torque exceeds the magnetic resistance, the magnet 22B slides sufficiently to allow the lever 72 to pass over the ridge 70 and thus allow the magnets 22A and 22B to realign.

By modulating the size and shape of the ridge 70, a resistive force as perceived by the user may be modified.

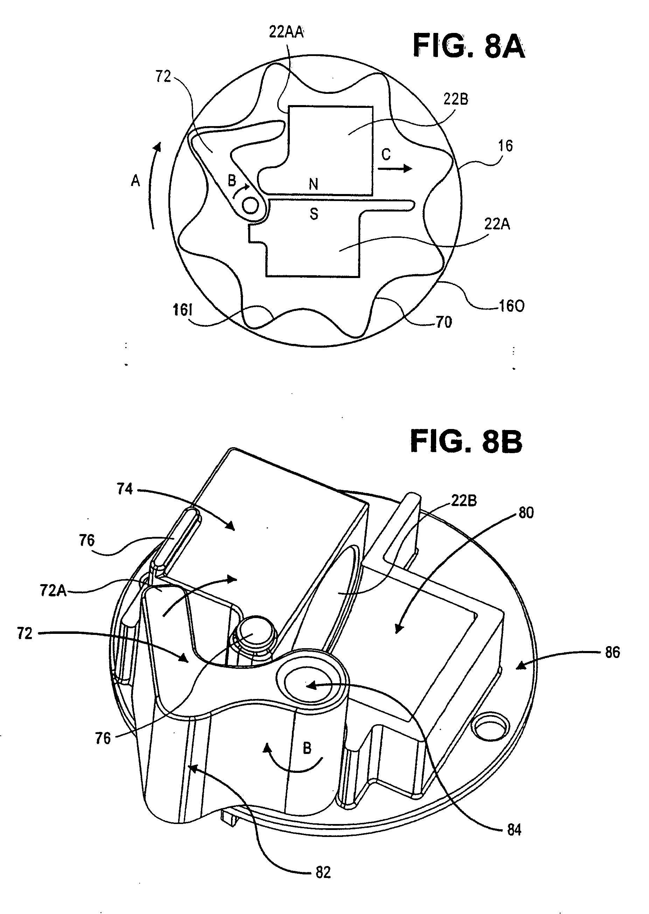

FIG. 8B is a perspective view of the torque-limiting device according to FIG. 8A. As shown in FIG. 8B, the magnet 22B may be secured within a housing 74. This housing 74 may include one or more tabs or pins 76 configured to slide within guide slots (not shown). To retain the magnet 22A, the torque-limiting device 10 may include a cradle 80 (upper half removed for clarity) configured to secure the magnet 22A (not shown).

Also shown in FIG. 8B, the lever optionally includes a radiused edge 82 to bear against the ridge 70. The lever 72 pivots via pivot point 84.

Therefore, the embodiments shown in FIGS. 8A and 8B include the torque-limiting device 10, shaft 18, housing 16, first magnet 22A, second magnet 22B and lever 72. The shaft 18 includes a distal end 18D to engage a fastener 12 and a proximal end 18P. The shaft 18 defines an axis of rotation X along which a rotation vector R is aligned towards the distal end 18D. The housing 16 is pivotally secured to the proximal end 18P. The housing 16 includes an outer surface 16O and an inner surface 161. The inner surface 161 includes at least one lug 70 protruding radially inwardly into the housing 16. The first magnet 22A is disposed within the housing 16. The first magnet 22A is secured to the proximal end 18P. The second magnet 22B is slidably secured to the proximal end 18P and juxtaposed in attractive cooperative alignment with the first magnet 22A. The second magnet 22B includes a magnet bearing surface 22BA. The first magnet 22A is urged towards alignment with the second magnet 22B with an attractive magnetic force 24. The lever 72 is pivotally secured to the proximal end 18P. The lever 72 has a lug bearing surface 82 to bear against the lug 70. The lever 72 has an arm 72A to bear against the magnet bearing surface 22BA. A clockwise external torque applied to the outer surface 16O urges the lug 70 to bear against the lug bearing surface 82 and urges the lever 72 to pivot clockwise. The arm 72A bears against the magnet bearing surface 22BA and urges the second magnet 22B out of alignment from the first magnet 22A in response to the clockwise urging of the arm 72 and the rotation of the housing 16 relative to the shaft 18 is impeded until the first magnet 22A and the second magnet 22B laterally slide past each other by exceeding the attractive magnetic force 24 between them.

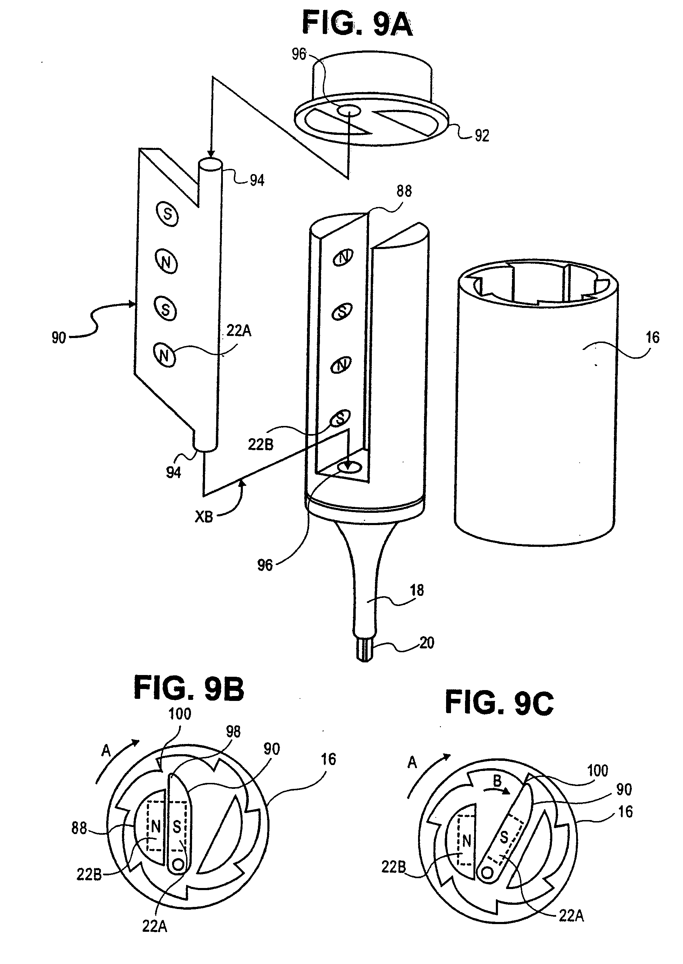

FIG. 9A is an exploded view of a torque-limiting device utilizing the magnetic alignments shown in FIG. 2C. As shown in FIG. 9A, the torque-limiting device includes a frame 88 and a pivoting holder 90. The frame 88 is configured to retain one or more magnets such as the magnet 22B. The pivoting holder 90 is configured to a magnetically attractive material or complimenting magnet such as the magnet 22A. In a particular example, the frame 88 may include 4 or more magnets and the pivoting holder 90 may include a matching number of magnets.

The frame 88 and pivoting holder 90 are operable to juxtapose the magnets 22A and 22B in cooperative alignment. The handle 16 is configured to slide over the frame 88 and pivoting holder 90. When assembled, the handle 16 and pivoting holder 90 may be retained by a cap 92. The pivoting holder 90 includes a pair of pins 94 configured to mate with a pair of bores 96 and rotate therein.

FIG. 9B is a top view of the torque-limiting device of FIG. 9A. As shown in FIG. 9B, the pivoting holder 90 includes a lip 98 configured to catch on a lug 100 as the handle 16 is rotated in direction A.

FIG. 9C is a top view of the torque-limiting device in a second configuration according to FIG. 9A. As shown in FIG. 9C, given sufficient torque supplied by rotation of the handle 16 in direction A, the magnetic attraction of the magnets 22A and 22B may be overcome and the pivoting holder 90 may be displaced in direction B. As the magnet 22A moves further from the magnet 22B, the magnetic attraction diminishes. At a predetermined point in the rotation of the handle 16, the lug 100 slides over and disengages from the lip 98. In response to disengagement and the magnetic attraction of the magnets 22A and 22B, the pivoting holder 90 is allowed to return to the initial conformation shown in FIG. 9B.

Therefore, the embodiments shown in FIGS. 9A, 9B and 9C include the torque-limiting device 10, shaft 18, first magnetic assembly 88, housing 16, and second magnetic assembly 90. The shaft 18 includes a distal end 18D to engage a fastener 12 and a proximal end 18P. The first magnetic assembly 88 is secured to the proximal end 18P. The first magnetic assembly 88 includes a first plurality of magnets 22As. The housing 16 is pivotally secured to the proximal end 18P. The housing 16 includes an outer surface 160 to receive an applied torque and the housing 16 includes an inner surface 161. The inner surface 161 includes at least one lug 100 protruding radially inwardly into the housing 16. The second magnetic assembly 90 is pivotally secured to the proximal end 18P. The second magnetic assembly 90 includes a second plurality of magnets 22Bs, an axis XB about which the second magnetic assembly 90 rotates, and a lip 98 offset from the axis XB. The second magnetic assembly 90 is juxtaposed in attractive cooperative alignment with the first magnetic assembly 88 to generate an attractive magnetic force between them. The rotation of the housing 16 relative to the shaft 18 urges the lug 100 to bear upon the lip 98 and rotate the second magnetic assembly 90 away from and out of alignment with the first magnetic assembly 88. The rotation of the housing 16 relative to the shaft 18 is impeded until the separation of the first magnetic assembly 88 and the second magnetic assembly 90 in response to exceeding the attractive magnetic force between 24 them.

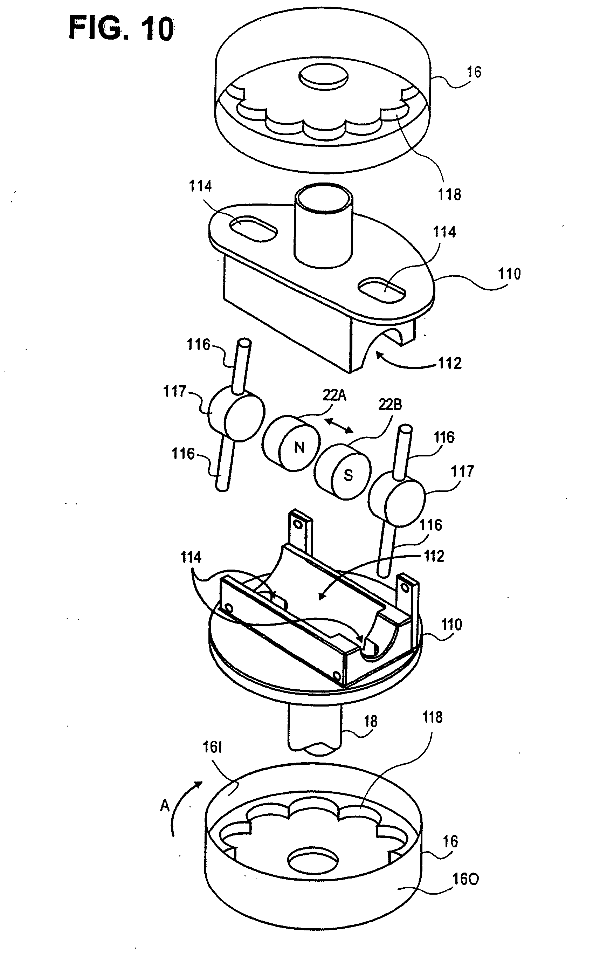

FIG. 10 is an exploded view of the torque-limiting device 10 of FIG. 2D. As shown in FIG. 10, the torque-limiting device according to this embodiment utilizes magnetic repulsion to generate the resistive force. In a particular example, the North pole or face of both the magnets 22A and 22B are aligned to face one another. The torque-limiting device includes an assembly 110 that is secured to the shaft 18. The assembly 110 includes a cradle, or race 112 to retain the magnets 22A and 22B in cooperative alignment. In general, the race 112 may include any suitable conformation to retain the magnets 22A and 22B in cooperative alignment. In addition, the race 112 facilitates movement of the magnets 22A and 22B with respect to one another while retaining cooperative alignment of the magnets 22A and-22B. The assembly 110 further includes slots 114 through which respective pins 116 may protrude. The pins 116 may be retained in stops 117 that are configured to secure the magnets 22A and 22B within the race 112.

The pins 116 are configured to engage one or more cams 118. In a particular example, a matching and indexed set of the cams 118 may be disposed above and below the assembly 110 and may be configured to drive the pins inward in unison. As the handle 16 is rotated, these cams 118 urge the pins 116 inward. This action, in turn, urges the magnets 22A and 22B toward one another. As the magnets 22A and 22B get closer, the magnetic repulsion increases. Accordingly, by modulating the displacement or shape of the cams 118, the torqueₘₐₓ may be controlled. In addition, modifying the size, shape, magnetic material, degree of magnetism, and the like may also be utilized to adjust the torqueₘₐₓ.

Therefore, the embodiment shown in FIG. 10 includes the torque-limiting device 10, shaft 18, housing 16, race 112, first magnet 22A, and second magnet 22B. The shaft 18 includes the distal end 18D to engage the fastener 12 and the proximal end 18P. The shaft 18 defines the axis X of rotation along which the rotation vector R is aligned towards the distal end 18D. The housing 16 is pivotally secured to the proximal end 18P. The housing 16 includes an outer surface 160 and an inner surface 161. The inner surface 161 includes at least one cam 118 protruding radially inwardly into the housing 16. The race 112 is secured to the proximal end 18P. The race 112 is oriented perpendicularly relative to the shaft 18. The first magnet 22A is slidably disposed within the race 112. The first magnet 22A includes a bearing surface 116 to bear against the cam 118. The second magnet 22B is slidably disposed within the race 112 and juxtaposed in repulsive cooperative alignment with the first magnet 22A. The first magnet 22A is repulsed from the second magnet 22B with a repulsive magnetic force 24R that urges the bearing surface 116 against the cam 118 to impede rotation of the housing 16 relative to the shaft 18. An applied torque to the outer surface 16O urges the cam 118 to bear against the bearing surface 116 and urges the first and second magnets 22A and 22B towards the center of the race. The rotation of the housing 16 relative to the shaft 18 compels shaft 18 to rotate until the applied torque exceeds a predetermined limit which is when the bearing surfaces 116 reach the full extent of the cam displacement and fall back into the cam valley thereby permitting the magnets to return to their remote lacations.

In addition, the embodiments shown in FIGS. 3A to 10 include the torque-limiting device 10, a first end 18D to engage a fastener 12, a second end 16 to receive an applied torque, and a magnetic torque limiter to transmit the applied torque from the second end 16 to the first end 18D to rotate the first end 18D in conjunction with the second end 16 when the applied torque is less than a predetermined limit.

Furthermore, the embodiments shown in FIGS. 3A to 10 include the torque-limiting device 10, shaft means such as the shaft 18 or the like, handle means such as the handle 16 or the like, and magnetic torque-limiting means such as two or more magnetically active subunits cooperatively aligned to generate a magnetic force. The shaft means engages a fastener. The handle means receives an applied torque. The magnetic torque-limiting means couples the shaft means and handle means and magnetically limiting an amount of torque externally applied to the handle means from being transmitted to the fastener. The magnetic torque-limiting means is configured to transmit the applied torque from the handle means to the shaft means in response to the applied torque being less than a predetermined torque limit.

Conventional torque measuring devices are based on torsion bar design in which applied torque is indicated by a pointer and scale attached to a conventional torque measuring tool. The conventional measuring tool is subject to metal fatigue and wear and the torque range changes, requiring that they be returned for periodic adjustment and maintenance. The conventional devices are calibrated and adjusted mechanically with the help of an electronic load cell transducer. These devices are composed of complicated high precision components that are assembled by hand and easily damaged. As such, these conventional instruments are costly to produce and maintain and require a high selling price.

In various embodiments of the present invention, a torque sensing device including a magnetic assembly having two magnetic elements that are either mutually attracting or mutually repelling each other. The force of such a (finite) magnetic assembly or magnetic circuit will be constant. As the predetermined magnetic circuit force is constant then a displacement force or movement or pressure will be constant and repeatable. Interfering with the circuit by applying a counter force will yield a displacement factor in the physical proximity of the two magnet elements. This displacement can be indicated, measured and recorded. The magnetic force is essentially constant and of unchanging value. As such, the need for calibration and adjustment may be reduced or eliminated. This force can be measured by means of a gauss meter or measured by applying a force greater than the magnetic force this greater force may be in the form of a physical displacement of the relationship between the two magnets. Furthermore the displacing force may take the form of slipping the magnets apart or by pulling the two magnets apart along their common axis or by tipping or bending the magnets apart.

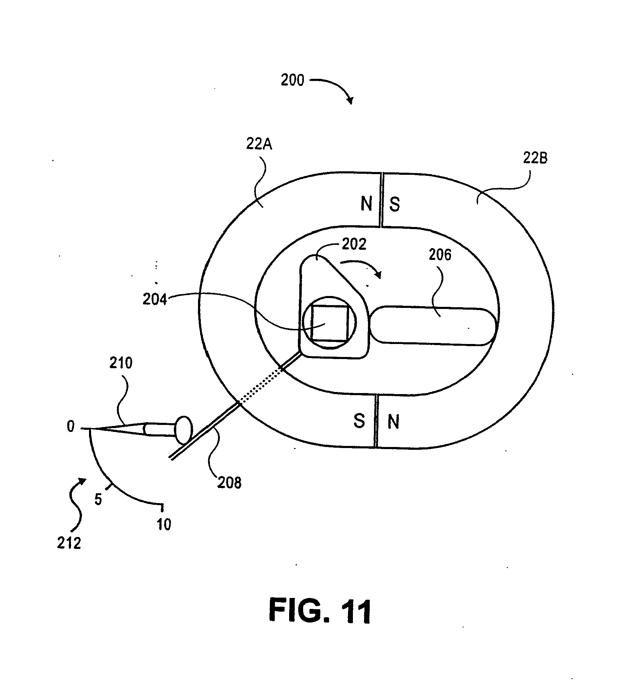

FIG. 11 is a simplified top view of a torque sensing device 200 according to the embodiment of FIG. 2A. In general, the torque measurement or sensing device 200 utilizes a magnetically attractive field and/or magnetically repelling field to measure torque. For example, the torque sensing device 200 may be configured to sense the torque produced by the torque-limiting device 10. As shown in FIG. 11, the torque sensing device 200 includes a cam 202 that is pivotally secured to the magnet 22A. The cam 202 is configured to rotate about an axis 204 and urge a follower 206 towards the magnet 22B. The torque sensing device 200 further includes a sweep arm 208 that is affixed to the cam 202. The sweep arm 208 is configured to bear against an indicator 210. In response to rotation of the cam 202, the sweep arm 208 is configured to move relative to the indicator 210 and rotate the indicator 210 accordingly along a gauge 212. Torque applied to the torque sensing device 200 may therefore be determined based on the indicator 210 progress along the gauge 212.

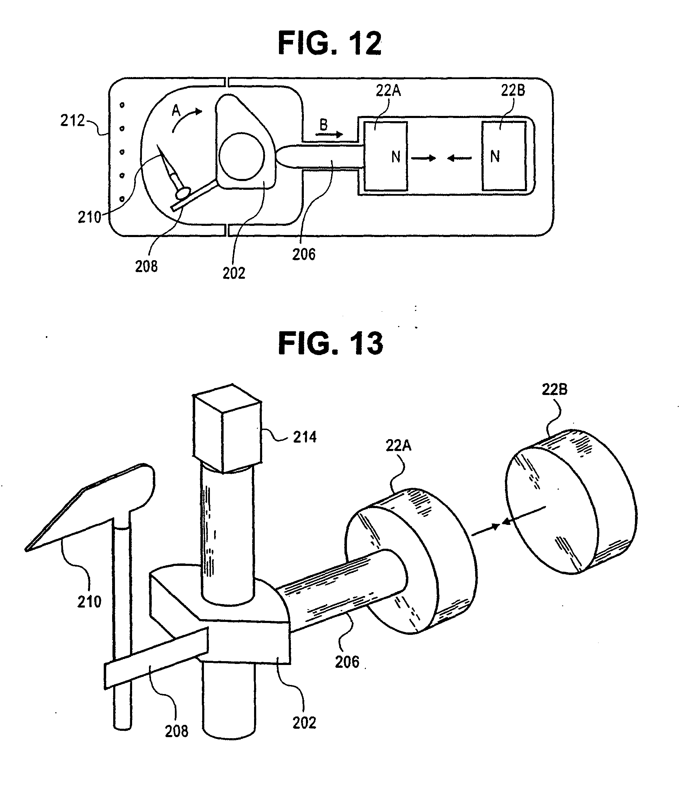

FIG. 12 is a top view of the torque sensing device 200 according to the embodiment of FIG. 2D. In general, the torque sensing device 200 includes two mutually repelling magnets aligned through the common magnetic axis established by their proximity to one another. The repelling force may be predetermined to exceed the torque force to be measured. As shown in FIG. 12, the magnets 22A and 22B are juxtaposed with respective North poles facing one another in cooperative alignment. When rotated in direction A, the cam 202 is configured to urge the follower 206 in direction B. This movement of the follower 206 urges the magnets 22A and 22B towards one another thus, increasing the magnetic repulsion. The indicator 210 is rotated in response to movement of the sweep arm 208 and indicates the torque level attained. Alternately, a pressure transducer or other such electro-mechanical sensor may be used to indicate torque. In another alternative, a magnet gauss meter may read the change in field waves and indicate torque. Furthermore, the torque sensing device 200 may include any suitable load sensor or display device. Suitable examples of load sensors and display devices include piezoelectric force/strain gauges, passive indicators, electronic readouts, potentiometers, LCD displays, and the like. As shown in FIG. 13, attached to the cam 202 is an adapter 214 suitable to attaching various torque-limiting tools such as the torque-limiting device 10.

In another embodiment, the torque sensing device 200 may be secured to the torque-limiting device 10. In this embodiment, the torque applied to the fastener 12 may be sensed as the fastener 12 is installed in the substrate 14 as shown in FIG. 1.

FIGS. 14A and 14B are a cross sectional views A-A of the torque-limiting device 10 in a first and second, respectively, torque-limiting configuration. In general, as shown in FIG. 14A, the shaft magnet assembly 50 is aligned with the handle magnet assembly 52 while in the first torque liming configuration. As a result of the alignment, the torque limit is at a relatively maximum value. In general, as shown in FIG. 14B, the shaft magnet assembly 50 is relatively maximally misaligned with the handle magnet assembly 52 while in the second torque liming configuration. As a result of the relatively maximally mis-alignment, the torque limit is at a relatively minimum value. 10. More particularly, in the example shown, the torque-limiting tool 10 includes 5 pairs of shaft magnet assemblies 50 and handle magnet assemblies 52. When in the second torque-limiting configuration, essentially ½ of the most proximal shaft magnet assembly 50 is unpaired with a handle magnet assembly 52 and essentially ½ of the most distal handle magnet assembly 52 is unpaired with a shaft magnet assembly 50. Therefore, in the second torque-limiting configuration, the torque limit of the torque-limiting tool 10 is substantially the same as if the torque-limiting tool included 4 pairs of shaft magnet assemblies 50 and handle magnet assemblies 52. To facilitate adjustment of the mis-alignment, the torque-limiting device 10 includes a thrust bearing 300. The thrust bearing 300 includes an adjustment ring 302 affixed to the handle magnet assembly 52 and an inner sleeve 304 that is configured to axially urge the shaft magnet assembly 50. The adjustment ring 302 threadedly engages the inner sleeve 304. By rotating the adjustment ring 302 relative to the inner sleeve 304, the torque limit of the torque-limiting tool 10 may be adjusted. To retain a set adjustment, the thrust bearing 300 includes a set screw 306 to threadedly engage a threaded bore 308.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A torque-limiting device comprising:
a first end to engage a fastener;
a second end to receive an applied torque; and
a magnetic torque limiter to transmit the applied torque from the second end to the first end to rotate the first end in conjunction with the second end when the applied torque is less than a predetermined limit.

2. The torque-limiting device according to claim 1, wherein the magnetic torque limiter further comprises a plurality of magnetically active materials juxtaposed in cooperative alignment.

3. The torque-limiting device according to claim 1, wherein the cooperative alignment of the plurality of magnetically active materials generates a magnetic circuit between the plurality of magnetically active materials.

4. A torque-limiting device comprising:
a shaft including a distal end to engage a fastener and a proximal end, the shaft defining an axis around which a clockwise rotation vector is aligned towards the distal end;
a housing pivotally secured to the proximal end, the housing including an outer surface and an inner surface, the inner surface including at least a first lug protruding radially inwardly into the housing and at least a second lug protruding radially inwardly into the housing;
a first magnet disposed within the housing, the first magnet being secured to the proximal end, the first magnet including a first bearing surface;
a second magnet juxtaposed in attractive cooperative alignment with the first magnet, the second magnet including a second bearing surface, wherein the first magnet is drawn towards the second magnet with an attractive magnetic force;
a first lever pivotally secured to the proximal end, the first lever having a first lever lug arm to bear against the first lug, the first lever having a first lever magnet arm to bear against the first bearing surface, wherein a clockwise external torque applied to the outer surface urges the first lug to bear against the first lever lug arm and urges the first lever to pivot clockwise, wherein the first lever magnet arm bears against the first bearing surface to urge the first magnet away from the second magnet in response to the clockwise urging of the first lever lug arm; and
a second lever pivotally secured to the proximal end, the second lever having a second lever lug arm to bear against the second lug, the second lever having a second lever magnet arm to bear against the second bearing surface, wherein the clockwise external torque applied to the outer surface urges the second lug to bear against the second lever lug arm and urges the second lever to pivot clockwise, wherein the second lever magnet arm bears against the second bearing surface to urge the second magnet away from the first magnet in response to the clockwise urging of the second lever lug arm and wherein rotation of the housing relative to the shaft is impeded until the first magnet and second magnet are separated by exceeding the attractive magnetic force between them.

5. The torque-limiting device according to claim 4, wherein the outer surface is configured to mate with an anatomically human hand.

6. A torque-limiting device comprising:
a shaft including a distal end to engage a fastener and a proximal end, the shaft defining an axis of rotation along which a rotation vector is aligned towards the distal end;
a housing pivotally secured to the proximal end, the housing including an outer surface and an inner surface, the inner surface including at least a first toothed region having teeth protruding radially inwardly into the housing and at least a second toothed region having teeth protruding radially inwardly into the housing;
a first magnet disposed within the housing, the first magnet being secured to the proximal end, the first magnet including a first toothed carrier;
a second magnet juxtaposed in attractive cooperative alignment with the first magnet, the second magnet including a second toothed carrier, wherein the first magnet is drawn towards the second magnet with an attractive magnetic force;
a first gear pivotally secured to the proximal end, the first gear having teeth to engage the first toothed region and the first toothed carrier, wherein a clockwise external torque applied to the outer surface urges the first toothed region to engage the first gear and clockwise rotate the first gear, wherein clockwise urging of the first gear urges the first magnet away from the second magnet; and
a second gear pivotally secured to the proximal end, the second gear having teeth to engage the second toothed region and the second toothed carrier, wherein the clockwise external torque applied to the outer surface urges the second toothed region to engage the second gear and clockwise rotate the second gear, wherein clockwise urging of the second gear urges the second magnet away from the first magnet and wherein rotation of the housing relative to the shaft is impeded until the first magnet and the second magnet are separated by exceeding the attractive magnetic force between them.

7. The torque-limiting device according to claim 6, wherein the cooperative alignment of the first and second magnets generates a magnetic circuit between the first and second magnets.

8. A torque-limiting device comprising:
a shaft including a distal end to engage a fastener and a proximal end;
a first magnetic assembly secured to the proximal end, the first magnetic assembly including a first plurality of magnets, each one of the first plurality of magnets having a North side adjacent to a South side of another one of the first plurality of magnets;
a housing pivotally secured to the proximal end, the housing including an outer surface to receive an applied torque; and
a second magnetic assembly secured to the housing, the second magnetic assembly including a second plurality of magnets, each one of the second plurality of magnets having a North side adjacent to a South side of another one of the second plurality of magnets, wherein each of the North and South sides of the second magnetic assembly is juxtaposed in attractive cooperative alignment with respective opposite South and North sides, respectively of, the first magnetic assembly to generate an array of attractive magnetic forces between the shaft and the housing.

9. The torque-limiting device according to claim 8, wherein rotation of the housing relative to the shaft is impeded until the applied torque exceeds a predetermined limit associated with the attractive magnetic force.

10. A torque-limiting device comprising:
a shaft including a distal end to engage a fastener and a proximal end, the shaft defining an axis of rotation along which a rotation vector is aligned towards the distal end;
a housing pivotally secured to the proximal end, the housing including an outer surface and an inner surface, the inner surface including at least a first toothed region having teeth protruding radially inwardly into the housing and at least a second toothed region having teeth protruding radially inwardly into the housing;
a first magnet disposed within the housing, the first magnet being secured to the proximal end, the first magnet including a first toothed carrier;
a second magnet juxtaposed in attractive cooperative alignment with the first magnet, the second magnet including a second toothed carrier, wherein the first magnet is aligned with the second magnet with an attractive magnetic force between them;
a first gear pivotally secured to the proximal end, the first gear having teeth to engage the first toothed region and the first toothed carrier, wherein a clockwise external torque applied to the outer surface urges the first toothed region to engage the first gear and clockwise rotate the first gear, wherein clockwise urging of the first gear urges the first magnet to laterally slide out of alignment with the second magnet; and
a second gear pivotally secured to the proximal end, the second gear having teeth to engage the second toothed region and the second toothed carrier, wherein the clockwise external torque applied to the outer surface urges the second toothed region to engage the second gear and clockwise rotate the second gear, wherein clockwise urging of the second gear urges the second magnet to laterally slide out of alignment with the first magnet and wherein rotation of the housing relative to the shaft is impeded until the first magnet and the second magnet laterally slide past each other by exceeding the attractive magnetic force between them.

11. The torque-limiting device according to claim 10, wherein the attractive magnetic force determines a maximum amount of torque that may be transmitted to the fastener from the housing.

12. A torque-limiting device comprising:
a shaft including a distal end to engage a fastener and a proximal end, the shaft defining an axis of rotation along which a rotation vector is aligned towards the distal end;
a housing pivotally secured to the proximal end, the housing including an outer surface and an inner surface, the inner surface including at least one lug protruding radially inwardly into the housing;
a first magnet disposed within the housing, the first magnet being secured to the proximal end;
a second magnet slidably secured to the proximal end and juxtaposed in attractive cooperative alignment with the first magnet, the second magnet including a magnet bearing surface, wherein the first magnet is urged towards alignment with the second magnet with an attractive magnetic force; and
a lever pivotally secured to the proximal end, the lever having a lug bearing surface to bear against the lug, the lever having an arm to bear against the magnet bearing surface, wherein a clockwise external torque applied to the outer surface urges the lug to bear against the lug bearing surface and urges the lever to pivot clockwise, wherein the arm bears against the magnet bearing surface and urges the second magnet out of alignment from the first magnet in response to the clockwise urging of the arm and wherein rotation of the housing relative to the shaft is impeded until the first magnet and the second magnet laterally slide past each other by exceeding the attractive magnetic force between them.

13. The torque-limiting device according to claim 12, wherein the attractive magnetic force determines a maximum amount of torque that may be transmitted to the fastener from the housing.

14. A torque-limiting device comprising:
a shaft including a distal end to engage a fastener and a proximal end;
a first magnetic assembly secured to the proximal end, the first magnetic assembly including a first plurality of magnets;
a housing pivotally secured to the proximal end, the housing including an outer surface to receive an applied torque and the housing including an inner surface, the inner surface including at least one lug protruding radially inwardly into the housing; and
a second magnetic assembly pivotally secured to the proximal end, the second magnetic assembly including a second plurality of magnets, an axis about which the second magnetic assembly rotates, and a lip offset from the axis, wherein the second magnetic assembly is juxtaposed in attractive cooperative alignment with the first magnetic assembly to generate an attractive magnetic force between them, wherein rotation of the housing relative to the shaft urges the lug to bear upon the lip and rotate the second magnetic assembly away from and out of alignment with the first magnetic assembly, wherein rotation of the housing relative to the shaft is impeded until the separation of the first magnetic assembly and the second magnetic assembly in response to exceeding the attractive magnetic force between them.

15. The torque-limiting device according to claim 14, wherein the attractive magnetic force determines a maximum amount of torque that may be transmitted to the fastener from the housing.

16. A torque-limiting device comprising:
a shaft including a distal end to engage a fastener and a proximal end, the shaft defining an axis of rotation along which a rotation vector is aligned towards the distal end;
a housing pivotally secured to the proximal end, the housing including an outer surface and an inner surface, the inner surface including at least one lug protruding radially inwardly into the housing;
a race secured to the proximal end, the race being oriented perpendicularly relative to the shaft;
a first magnet slidably disposed within the race, the first magnet including a bearing surface to bear against the lug; and
a second magnet slidably disposed within the race and juxtaposed in repulsive cooperative alignment with the first magnet, wherein the first magnet is repulsed from the second magnet with a repulsive magnetic force that urges the bearing surface against the lug to impede rotation of the housing relative to the shaft, wherein an applied torque to the outer surface urges the lug to bear against the bearing surface and urges the first magnet towards the second magnet and wherein rotation of the housing relative to the shaft is impeded until the applied torque exceeds a predetermined limit.

17. The torque-limiting device according to claim 16, further comprising:
a second lug protruding radially inwardly into the housing;
a second bearing surface to bear against the second lug and urge the second magnet towards the first magnet in response to the applied torque.

18. A torque-limiting device comprising:
shaft means for engaging a fastener;
handle means for receiving an applied torque; and
a magnetic torque-limiting means coupling the shaft means and handle means and magnetically limiting an amount of torque externally applied to the handle means from being transmitted to the fastener, the magnetic torque-limiting means being configured to transmit the applied torque from the handle means to the shaft means in response to the applied torque being less than a predetermined torque limit.

19. The torque-limiting device according to claim 18, wherein the magnetic torque-limiting means is configured to allow the shaft means to rotate relative to the handle means in response to the applied torque being greater than the predetermined torque limit.

20. A torque meter comprising:
a cam to receive an applied torque; and
a magnetic coupling, the magnetic coupling including a pair of magnetically active components that are urged together by an attractive magnetic force, wherein the cam is configured to urge the pair of magnetically active components apart in response to the applied torque.

21. The torque meter according to claim 20, further comprising:
an indicator to indicate the applied torque in response to rotation of the cam relative to the magnetic coupling.
